# EUROPEAN PATENT APPLICATION

(11) **EP 1 481 706 A1**
(43) Date of publication of application: **01.12.2004**
(21) Application number: 04253181.4
(22) Date of filing: 28.05.2004
(51) Int. Cl.: A61N 1/05

(54) **Fixation of a left heart medical lead in the coronary sinus**

(30) Priority: 30.05.2003 US 449972
(71) Applicant: PACESETTER, INC., Sylmar, CA 91392-9221 (US)
(72) Inventor: Morgan, Kevin L., Simi Valley California 93063 (US); Chitre, Yougandh, Valencia California 91355 (US); Helland, John R., Saugus California 91350 (US); Bornzin, Gene A., Simi Valley California 93065 (US); Doan, Phong D., Stevenson Ranch California 91381 (US)
(74) Representative: Rees, David Christopher

(57) **Abstract**

An implantable lead (10) is provided for delivering electrical therapy. The lead (10), suitable for placement and passive anchoring in a vessel (84) within the coronary sinus region (82) of a patient's heart (70), comprises a lead body (14) having a distal end (16) carrying at least one electrode (32) electrically connected to a terminal contact on a proximal end (18) of the lead body (14), the lead body (14) further including an insulative, tubular housing (20). A longitudinally extending coil member (40) is contained within the housing (20), the housing (20) and the coil member (40) having distal ends provided with corresponding, substantially matching preformed bends (46), the distal end of the housing (20) being thereby adapted to exert a sufficient bias against the inner wall (86) of the vessel (84) within which the lead (10) is placed to stabilize the lead (10) in the vessel (84).

## Description

The present invention relates generally to body implantable stimulation and sensing leads. More particularly, the invention relates to body implantable stimulation and sensing leads designed to be securely placed intravenously in the coronary sinus region and into the vein(s) of the heart to provide pacing, sensing and/or cardioversion/defibrillation of the left atrium and/or the left ventricle.

The advantages of providing pacing, sensing and/or shock therapies to both the right and left heart chambers are well established. For example, in four chamber pacing systems, multiple leads, typically bipolar leads, are positioned for both pacing and sensing the respective heart chambers. On the right side, implantable stimulation/sensing leads are typically positioned directly in the right chambers of the heart, that is, in the right atrium and/or ventricle, via the superior vena cava (SVC). Passive fixation of a conventional cardiac pacing lead in the right chambers of the heart employ means such as tines, fins, or the like, to anchor or stabilize the distal end of the lead and thereby resist micro and gross displacements or dislodgment of the lead.

To provide left side stimulation and sensing, leads are usually transvenously implanted in the coronary sinus region, for example, in one of the coronary veins such as the great cardiac vein or the left posterior ventricular (LPV) vein proximate the left ventricle of the heart. Such placement avoids the risks associated with implanting a lead directly within the left ventricle which can increase the potential for the formation of blood clots which may become dislodged and then carried to the brain where even a small embolism could cause a stroke. (As used herein, the phrase "coronary sinus region" refers to the coronary sinus, great cardiac vein, left marginal vein, left posterior ventricular (LPV) vein, middle cardiac vein, and/or small cardiac vein or any other coronary vein accessible by way of the coronary sinus.)

The tip electrode of a lead implanted in a vein in the coronary sinus region can pace and sense left side ventricular activity. When such a lead includes a second electrode proximal of the tip electrode and residing in the coronary sinus above the left ventricle closely adjacent to the left atrium of the heart, pacing and sensing of left atrial activity is also made possible. Moreover, the lead may include one or more electrodes for the delivery of electrical shocks for terminating tachycardia and/or fibrillation. Such cardioverting/defibrillating electrodes may be used by themselves or may be combined with pacing and/or sensing electrodes.

While a pacing/sensing lead with tines or fins can be wedged in the apex of the right ventricle (RV) for stable fixation, the anchoring or stabilizing of a lead in a vein such as the coronary sinus poses more of a challenge because the lead is oriented essentially parallel with the walls of the vein. Like the posterior vein of the left ventricle, distal coronary vessel tributaries have small diameters. The leads placed in these vessels should track well and have a small diameter so they may be placed in distal vessels. The electrode should have intimate contact with the tissue and it should not dislodge. A lead having a diameter smaller than that of the vessel is likely to move easily within the vessel and may not be adequately affixed which results in displacement or dislodgment of the lead.

Leads particularly adapted for left side coronary venous placement may have precurved distal ends to aid in holding the distal end portion of the lead in place within a coronary vein by virtue of the frictional or bias forces exerted by the precurves against the wall of the vein. Examples of such leads are disclosed in U.S. patents 5,925,073; 5,387,233; and 6,129,750. The precurves are imparted to the distal ends of these leads by preforming either a coil within the polymer housing of the lead or the polymer housing itself. However, the weak normal forces exerted on the vessel walls by such preformed features in either the coil or the housing provide only a limited degree of lead fixation. Hence, robust fixation of a lead in a coronary vessel has been difficult to achieve.

Accordingly, there remains a need for a left side pacing/sensing lead that provides stable fixation along the vessel walls so as to alleviate or eliminate the high incidence of micro and gross lead displacements or dislodgment.

In accordance with one specific, exemplary embodiment of the invention, there is provided an implantable lead suitable for placement and passive anchoring in a vessel within the coronary sinus region of a patient's heart. The lead comprises a lead body including an insulative, tubular housing and having a distal end carrying at least one electrode electrically connected to a terminal contact on a proximal end of the lead body. The lead further comprises a longitudinally extending coil member contained within the lead body housing. The housing and the coil member have distal ends provided with corresponding, substantially matching preformed bends, the distal end of the housing being thereby adapted to exert a sufficient bias against the inner wall of the vessel within which the lead is placed to stabilize the lead in said vessel.

The foregoing and other objects, features and advantages of the invention will be evident to those skilled in the art from the detailed description below, taken together with the accompanying drawings, in which:
**FIG. 1** is a side elevation view of a unipolar pacing/sensing lead in accordance with a first embodiment of the present invention;
**FIG. 2** is transverse cross sectional view of the lead of **FIG. 1** as seen along the line 2-2 in **FIG. 1;**
**FIG. 3** is an axial cross sectional view of the lead of **FIG. 1** as seen along the line 3-3 in **FIG. 2;**
**FIG. 4** comprises juxtaposed side elevation views of the distal ends of the polymer housing and the coil conductor of the lead of **FIG. 1;**
**FIG. 5** is a perspective view of the anterior portion of a human heart showing the distal end of the lead of **FIG. 1** implanted in the coronary sinus region thereof;
**FIG. 6** is a side elevation view of a bipolar pacing/sensing lead in accordance with a second embodiment of the present invention;
**FIG. 7** is a transverse cross sectional view of the lead of **FIG. 6** as seen along the line 7-7 in **FIG. 6**;
**FIG. 8** is an axial cross sectional view of the lead of **FIG. 6** as seen along the line 8-8 in **FIG. 7**;
**FIG. 9** is a side elevation view of a bipolar pacing/sensing lead in accordance with a third embodiment of the present invention comprising a multilumen insulative housing;
**FIG. 10** is a transverse cross section view of the lead of **FIG. 9** as seen along the line 10-10 in **FIG. 9;** and
**FIG. 11** is an axial cross section view of the lead of **FIG. 9** as seen along the line 11-11 in **FIG. 10.**

The contexts in which the invention is shown and described herein, that is, specific unipolar and bipolar passive pacing and sensing leads, are illustrative only; it will be understood by those skilled in the art that the invention is equally applicable to a wide variety of unipolar, bipolar, multipolar and other body implantable tissue stimulating leads, including leads having passive fixation tines or fins and leads comprising active, screw-in fixation electrodes in the form of extendable helices.

In this description, the term "distal" refers to a direction toward, or a position closer to, the active or stimulating surface of the tip electrode, and the term "proximal" refers to a direction toward, or a position closer to, the end of the lead assembly that is adapted to be connected to the pulse generator.

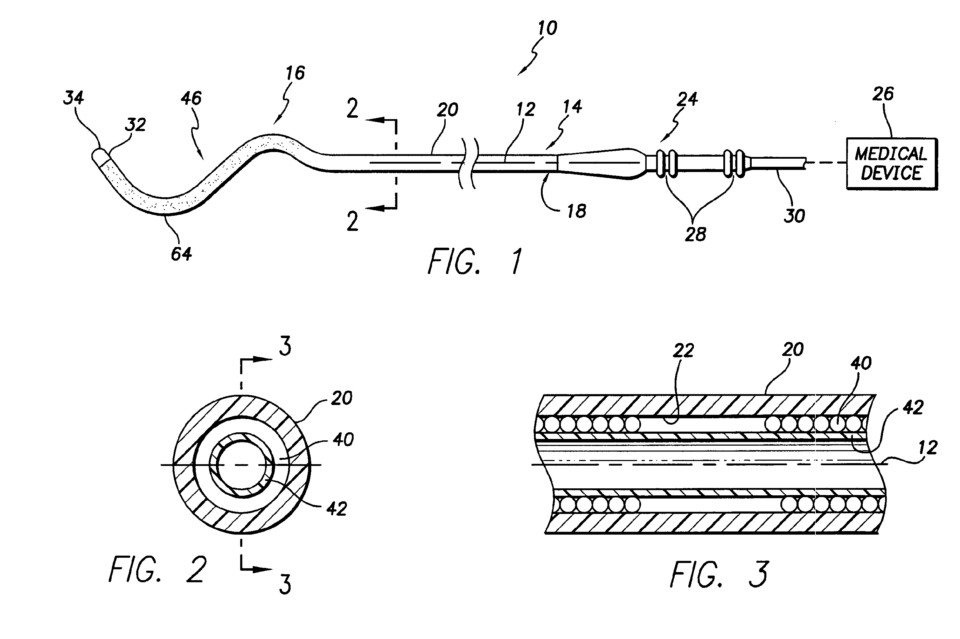

Referring now to **FIGS. 1-4**, there is shown in simplified form a first, specific, exemplary embodiment of the invention comprising a unipolar, intravenous pacing and sensing lead 10 having a longitudinal central axis 12. The lead 10 includes a lead body 14 comprising a distal end 16 and a proximal end 18. The lead body 14 is covered by a flexible, tubular insulative housing or sheath 20 having an interior surface 22 and made of a material such as polyurethane, silicone rubber, or similar biocompatible, biostable elastomeric polymer. By way of example and not limitation, the outside diameter of the lead body 14 may range from about 0.66 mm (0.026 inch) (2F) to about 2.3 mm (0.091 inch) (7F). In accordance with one form of the lead of the invention, the lead body 14 may be isodiametric, that is, the outside diameter of the lead body may be the same throughout its entire length. Further, the lead body may have a lubricious coating along most or all of its length to facilitate its movement through a left heart delivery introducer.

For left side placement, the distal end 16 of the lead body preferably has a length corresponding to the coronary sinus and its associated coronary vessels overlying the left side of the heart, ranging from approximately 4cm to approximately 20cm and preferably from about 6cm to about 10cm.

The proximal end 18 of the lead carries a connector assembly 24 adapted to be received by a socket or receptacle in a medical device 26 such as a pacemaker or implantable cardioverter/defibrillator (ICD). For this purpose, the connector assembly 24 includes longitudinally spaced sets of annular ribs or seals 28 for engaging the wall of the medical device receptacle so as to seal the receptacle against the entry of body fluids. The connector assembly 24 includes an electrical connector pin 30 adapted to engage a terminal contact within the receptacle of the medical device.

The distal end 16 of the lead carries a tip electrode 32 for performing unipolar pacing, sensing and/or cardioversion/defibrillation. The tip electrode 32 may be made of a platinum-iridium alloy or similar biostable, biocompatible, low polarization, conductive material. In the preferred embodiment, the platinum-iridium alloy has a composition of about 90% platinum and about 10% iridium by weight. Equivalent conductive materials such as stainless steel, an MP35N alloy, platinum, titanium, and platinum and titanium alloys, all well known in the pacing art, may be used.

The tip electrode 32 has a distal extremity defining an active exterior electrode surface 34. The active surface of the tip electrode may be covered with a coating of titanium nitride, platinum black, carbon black, iridium oxide or similar known materials for reducing electrode polarization, to provide Autocapture™ compatibility, and to present a roughened surface adapted to promote tissue ingrowth to help prevent dislodgement of the tip electrode. The area of the stimulating or active tip electrode surface preferably ranges from about 1.0 mm² to about 10.0 mm², with a preferred area range of about 3.0 mm² to about 5.0 mm², for providing a pacing impedance in the range of about 500 ohms to about 1,000 ohms.

The lead body 14 encloses a flexible, elongated electrical conductor 40 typically in the form of an elongated coil fabricated of an MP35N alloy or other suitable electrically conductive material. The coil conductor 40 has a proximal extremity electrically connected to the connector pin 30 and a distal end electrically connected, for example, by a laser weld, to the tip electrode 32. The elongated electrical conductor 40 transmits electrical signals bidirectionally between the tip electrode 32 and the medical device 26.

As is well known in the art, the coil conductor 40 may be multifilar for increased tensile strength and redundancy to provide continued stimulation and sensing in the event of failure of one of the conductor filaments.

The connector pin 30 on the proximal end of the lead body is preferably hollow so that in accordance with well known implantation techniques, a stylet or guide wire may be passed through the hollow connector pin and through the central passage or lumen of the coil conductor 40 to maneuver the distal end 16 of the lead body to place the tip electrode 32 at a desired location in the heart with the aid of an imaging means such as a fluoroscope. A low friction liner 42 of PTFE or the like may be provided within the lumen of the coil conductor to facilitate passage of the stylet or guide wire.

In accordance with the present invention, the distal end 16 of the lead body includes passive fixation means to securely anchor or stabilize the distal end portion within a target vessel of the coronary sinus region. The passive fixation or anchoring means preferably comprises one or more preformed, matching bends 46 manufactured into the distal ends of both the insulative housing and the coil conductor enclosed therein. The bends 46 may take various shapes, including but not limited to sinuous, S-shaped and spiral.

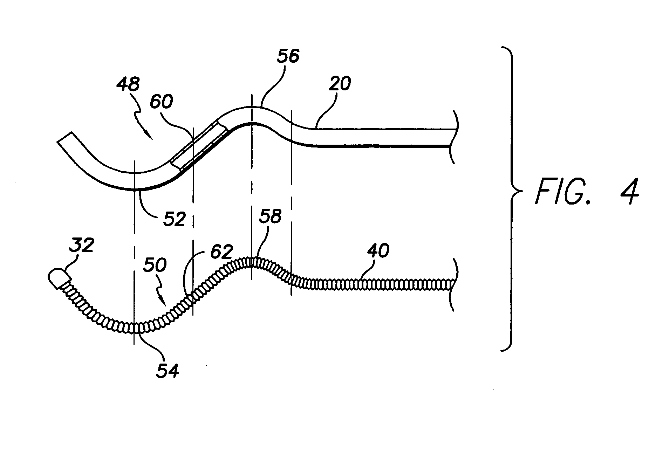

More specifically, with reference to **FIG. 4**, the insulative housing 20 has a distal end 48 that is preformed so as to have a shape that is substantially identical to that of a preformed distal end 50 of the coil conductor 40. By way of example, the distal ends 48 and 50 have matching S-shaped bends.

The preformed shapes of the corresponding distal ends of the housing 20 and the conductor coil 40 match so that the outer extremities 52 and 56 as well as the inflection point 60 of the bends in the housing 20 are in alignment or in phase with the corresponding outer extremities 54 and 58 and inflection point 62, respectively, of the bends in the coil 40 when the housing and the coil are juxtaposed as shown in **FIG. 4**. Preferably, the S-shaped bends in the distal ends of the housing and the coil are oriented so that they lie in a common plane, that is, the plane of the drawing sheet showing **FIG. 4**.

The combination of the preformed distal end 48 of the housing 20 and the matching preformed distal end 50 of the coil conductor 40 substantially increases the biasing force between the distal end 16 of the lead body and the inner vessel wall so as to enhance the anchoring or stabilizing of the lead.

Thus, when the distal end 16 of the lead body 14 is in place within a coronary vessel there will be firm biased contact between the bends and the inner wall of the target vessel so as to create frictional forces sufficient to wedge or anchor the lead and prevent its displacement or dislodgment.

It will be evident that the number of bends formed in the housing may be different than the number of bends formed in the coil conductor. So long as the bends that are common to both the housing and the coil conductor match, the advantages of the invention will be realized.

When the housing 20 is fabricated of polyurethane, the distal end 48 thereof may be appropriately shaped by heat treating the distal end and allowing thermal setting thereof in accordance with techniques known in the art. When the housing 20 is made of silicone rubber, the desired shape of the distal end of the housing may be achieved by, for example, injection molding, again in a manner well known in the art.

Preforming of the conductor coil 40 can be achieved by means of known heat treatment or equivalent processes.

It will be evident that the connector pin 30 and the tip electrode 32 may be electrically connected by means of a separate electrical cable or coil conductor (not shown) and that in such a case the coil 40 may simply comprise a conduit for a lead positioning stylet or guide wire. Further, there may be provided in combination with the preformed distal end shape, one or more soft, flexible protuberances (not shown) on the distal end of the housing that also tend to wedge the distal end of the lead body in the target coronary vessel. Such supplementary passive fixation means enhance the bias of the distal end of the lead body against the vessel wall.

The passive fixation means can further include texturization 64 of the distal end of the lead body housing to promote rapid blood clotting and resulting fibrotic tissue growth about the distal end portion to further aid in anchoring that portion in place. Such texturization may be formed by grit blasting or abrading the outer surface of the housing.

In one approach to the delivery of the lead to the coronary sinus and/or coronary veins, a "left heart lead delivery" work station or long percutaneous lead introducer adapted to reach into the coronary sinus is used to deliver the lead. The distal portion of the introducer may be curved with various single or compound curves to allow for ease in advancing the introducer through the coronary sinus os and into the coronary sinus. The inserted introducer provides a conduit facilitating and supporting the placement of the lead in the coronary sinus and ultimately into the more distal cardiac veins within the coronary sinus region.

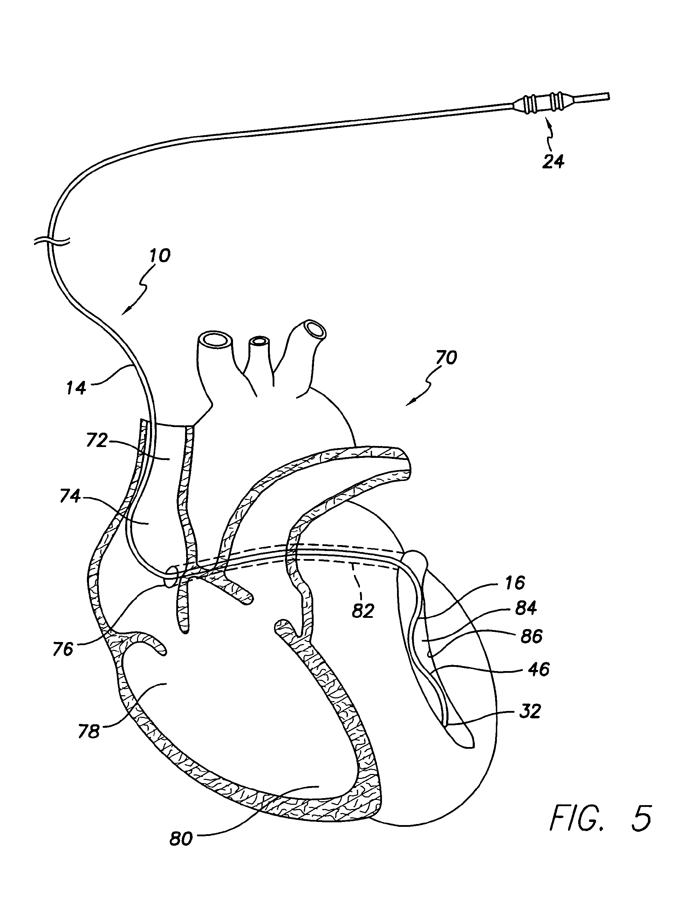

FIG. 5 is a diagrammatic, perspective view of the anterior portion of a human heart 70 showing the superior vena cava (SVC) 72, the right atrium 74, the coronary sinus ostium or os 76, the right ventricle 78 including the apex 80 thereof, the coronary sinus 82 and the left posterior ventricular (LPV) vein 84. The LPV vein 84 is one of a number of coronary veins accessible via the coronary sinus which, as indicated, are among the vessels residing in the coronary sinus region.

**FIG. 5** shows a lead body 14 in accordance with the above-described embodiment implanted in the coronary sinus region. In this particular example, the tip electrode 32 has been placed within the LPV vein 84 in contact with the wall 86 thereof. It will further be seen that the bends formed in the distal end 16 of the lead body have assumed their sinuous shape so as to be biased against the wall of the LPV vein thereby preventing displacement or dislodgment of the distal end of the lead body.

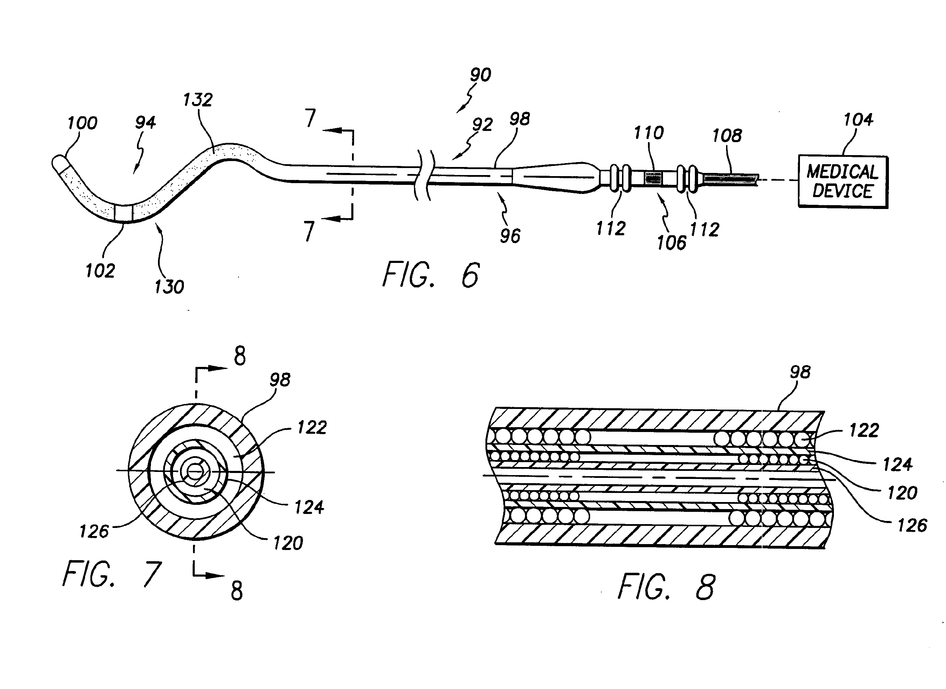

With reference to **FIGS. 6-8**, there is shown a bipolar endocardial pacing and sensing lead 90 in accordance with another preferred embodiment of the present invention. The lead 90 includes a lead body 92 comprising a distal end 94 and a proximal end 96. The lead body is covered by a tubular sheath or housing 98 made of an insulating, biocompatible, biostable material, preferably silicone rubber or polyurethane or a combination thereof. For left side placement, the distal end 94 portion of the lead body preferably has a length corresponding to the coronary sinus and its associated coronary vessels overlying the left side of the heart, ranging from approximately 4cm to approximately 20cm and preferably from about 6cm to about 10cm.

The distal end of the lead body 92 incorporates a tissue stimulating tip electrode 100. Disposed proximally of the tip electrode 100 along the distal end of the lead body is a ring electrode 102 for sensing electrical impulses produced by the heart tissue. It is desirable to have the distance between the tip and ring electrodes sufficiently small to allow both of these electrodes 100 and 102 to be placed in a target coronary vessel such as the LPV vein. Such placement of the electrodes ensures achieving electrical capture of the left ventricle, "electrical capture" being defined as the successful depolarization and contraction of a cardiac chamber, for example, the atrium or ventricle, in response to an electrical stimulation pulse generated by an implantable device such as a pacemaker or an implantable cardioverter/defibrillator(ICD). Other electrode configurations can be employed. For example, an alternate arrangement may include the use of two ring electrodes for sensing electrical signals generated by the heart. The kind of electrode configuration used will depend on the particular application and accordingly any electrode configuration known in the art (for example, pacing/sensing electrodes or defibrillation electrodes or any combination thereof at the lead tip or adjacent to the tip) may be utilized. With the electrode configuration shown in **FIG. 6**, an implantable medical device 104, such as a pacemaker or ICD, is connected to the tip and ring electrodes to perform bipolar sensing, pacing and/or cardioversion/defibrillation of the left ventricle through the coronary sinus region of the heart.

The proximal end 96 of the lead body 92 incorporates a connector assembly 106 for coupling the lead body to the medical device 104. The connector assembly 106 includes a hollow or tubular connector pin 108 electrically coupled to the tip electrode 100 and a ring terminal contact 110 electrically coupled to the ring electrode 102. As in the first embodiment, the connector assembly may include spaced sets of seals 112, and the lead body may be isodiametric with a diameter of, for example, 0.026 inch (2F) to 0.091 inch (7F) and have a lubricious coating on its outer surface.

The lead body 92 may accommodate various combinations of electrical coil and/or cable conductor combinations including, for example, a coil conductor and a cable conductor connected for bipolar operation, a pair of coaxial coils, multilumen combinations of coils and coils and cables, and so forth. The use of at least one coil conductor within at least the distal end of the lead body provides that end with greater flexibility further facilitating its maneuvering around sharp bends and corners in the coronary venous vasculature, while also forming one of the components of the passive fixation means of the present invention.

In the embodiment of **FIGS. 6-8**, a pair of concentric or coaxial coil conductors 120 and 122 with insulation 124 in between are carried within the insulative housing 98. The inner coil conductor 120 connects the pin terminal 108 on the connector assembly with the tip electrode 100, while the outer coil conductor 122, somewhat shorter than the inner conductor, connects the ring terminal contact 110 with the ring electrode 102. A low friction liner 126 of PTFE or the like may be provided within the inner coil conductor to facilitate passage of a stylet or guide wire for delivering and steering the distal end of the lead body during implantation. Alternatively (not shown), the liner may be in a separate lumen of the lead body. To reduce the outer diameter of the lead body, the individual coil conductors may be insulated and instead of being concentric, they may be interleaved and wound on the same diameter.

As in the first embodiment, the distal end of the lead body includes passive fixation means to help stabilize the distal end portion within a target vessel of the coronary sinus region. As before, the passive fixing or stabilizing means preferably comprises with one or more preformed bends 130 having, for example, an S-shaped or other sinuous configuration manufactured into both the distal end of the lead body insulative housing 98 and the distal end of at least one of the coil conductors 120, 122. Other preformed shapes, for example, spirals, humps, and the like, also may be used. In the specific, preferred embodiment of the invention shown in FIGS. 6-8, the distal ends of both the housing and either one or both of the coil conductors 120, 122 have matching bends as shown in **FIG. 4** and as already described in connection with the first embodiment. Accordingly, when the distal end 94 of the lead body 92 is in place within a coronary vessel, the combined forces exerted by the preformed housing and associated coil conductor(s) assure that there will be firm, biased contact between the bends and the inner wall of the target vessel so as to create sufficient frictional forces to securely wedge and stabilize the lead to prevent its displacement or dislodgment relative to the vessel wall.

As before, the passive fixation means may comprise in combination with the bends, one or more soft, flexible protuberances (not shown) on the outer surface of the distal end of the housing and/or texturization 132 of the distal end of the lead body.

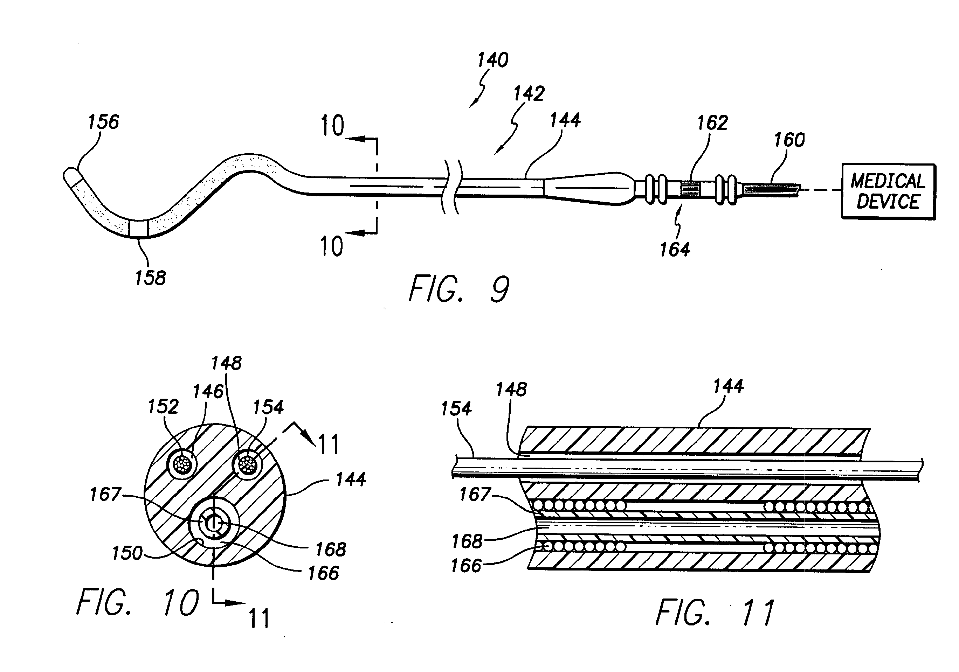

It will be evident that a lead body housing for use with the present invention may have various cross-sectional configurations. Thus, in accordance with yet another embodiment of the invention shown in **FIGS. 9-11**, there is provided a bipolar lead 140 having a lead body 142 including a tubular housing 144 of silicone rubber, polyurethane or similar polymer. The housing 144 in this specific embodiment comprises a trilumen structure defining lumens 146, 148 and 150 that may contain various combinations of electrical conductors and, optionally, a stylet of guide wire guiding coil. For example, in the specific embodiment shown, the lumens 146 and 148 carry braided cables 152 and 154, respectively, typically of an MP35N or MP35N/Ag alloy, preferably coated with an insulative layer of, for example, ETFE. These cable conductors form the electrical connections between the tip and ring electrodes 156 and 158 on the distal end of the lead body and corresponding terminal contacts 160 and 162 on a connector assembly 164 on the proximal end of the lead body. In the example shown, the third lumen 150 carries a longitudinally-extending coil 166 containing a low friction liner 167 of PTFE or the like defining a central passage 168 for guiding a stylet or a guide wire. Typically, the coil 166 will be electrically non-conductive but it will be apparent that the coil 166 may be electrically conductive, coupling a terminal contact on the connector assembly 164 with an electrode on the distal end of the lead body. Alternatively (not shown), the liner may be in a different lumen of the lead body

In accordance with the invention, to enhance the anchoring or fixation of the lead, the corresponding distal ends 144 of the housing and the guide coil 166 are preformed with substantially identical bends, as described in connection with the first embodiment and shown in **FIG. 4**. It will be apparent that a coil conductor may be substituted for either of the cable conductors 152 and 154. In that case, such coil conductor or conductors may also be preformed with bends or curves identical to those formed in the distal end of the housing.

## Claims

1. An implantable lead (10) for delivering electrical therapy, the lead being suitable for placement and passive anchoring in a vessel (84) within the coronary sinus (82) region of a patient's heart (70), the lead (10) comprising: a lead body (14) having a distal end (16) carrying at least one electrode (32) electrically connected to a terminal contact on a proximal end (18) of the lead body (14), the lead body (14) including an insulative, tubular housing (20); and a longitudinally extending coil member (40) within the housing (20), the housing (20) and the coil member (40) having distal ends provided with corresponding, substantially matching preformed bends (46), the distal end of the housing (20) being thereby adapted to exert a sufficient bias against the inner wall (86) of the vessel (84) within which the lead (10) is placed to stabilize the lead (10) in the vessel (84).

2. A lead as claimed in Claim 1, **characterised in that** the coil member (40) defines a passage for guiding a stylet or a guide wire.

3. A lead as claimed in Claim 1 or Claim 2, **characterised in that** the housing (20) comprises a multilumen structure, the coil member (40) occupying one of the lumens.

4. A lead as claimed in any preceding Claim, **characterised in that** an electrical conductor connects the at least one electrode (32) with the terminal contact, the electrical conductor occupying a second lumen in the housing (20).

5. A lead as claimed in Claim 4, **characterised in that** the electrical conductor comprises a coil conductor (120).

6. A lead as claimed in Claim 5, **characterised in that** both the coil member (40) and the electrical coil conductor (120) have matching preformed bends.

7. A lead as claimed in any preceding Claim, **characterised in that** the coil member (40) comprises an electrical conductor connecting the at least one electrode (32) with the terminal contact.

8. A lead as claimed in any preceding Claim, **characterised in that** the at least one electrode (32) comprises a tip electrode.

9. An implantable lead (90) for delivering electrical therapy, the lead (90) being suitable for placement in a vessel (84) within the coronary sinus region (82) of a patient's heart (70) and to be passively stabilized within the vessel (84), the lead (90) comprising: a lead body (92) having a distal end (94) carrying at least one electrode (100) electrically connected by a conductor (120) to a terminal contact (108) on a proximal end (96) of the lead body (92), the lead body (92) comprising a longitudinally extending, insulative housing (98) containing the conductor (120), both the housing (98) and the conductor (120) having substantially matching bends (130) preformed along the distal end (94) of the lead body (92), the distal end (94) of the lead body (92) being thereby adapted to exert a sufficient bias against the inner wall (86) of the vessel (84) to stabilize the lead (90) in said vessel (84).

10. A lead as claimed in Claim 8, **characterised in that** the at least one electrode (100) comprises a tip electrode and the conductor (120) comprises a coil conductor.

11. A lead as claimed in any preceding Claim, **characterised by** at least one ring electrode (102) electrically connected by a second conductor (120) to a second terminal contact (110) on the proximal end (96) of the lead body (92), the second conductor (120) being contained within the housing (98).

12. A lead as claimed in Claim 11, **characterised in that** the second conductor (120) is a coil conductor.

13. A lead as claimed in Claim 12, **characterised in that** the first mentioned conductor (122) and the second conductor (120) are disposed coaxially.

14. A lead as claimed in Claim 13, **characterised in that** the housing (98) and both the first mentioned conductor (122) and the second conductor (120) have substantially matching bends (130) preformed along the distal end (94) of the lead body (92).

15. A lead as claimed in any of Claims 11 to 14, **characterised in that** the housing (98) comprises a multilumen housing, the first mentioned conductor (122) and the second conductor (120) occupying separate lumens within the housing (98).

16. A lead as claimed in Claim 15, **characterised by** a guide coil defining a passage for a stylet or guide wire, the guide coil occupying a third lumen within the housing.
